# EUROPEAN PATENT APPLICATION

(11) **EP 2 228 003 A1**
(43) Date of publication of application: **15.09.2010**
(21) Application number: 09155168.9
(22) Date of filing: 13.03.2009
(51) Int. Cl.: A61B 1/05

(54) **Multifunctional endoscopic device and methods employing said device**

(71) Applicant: Blume, Jürgen, 69231 Rauenberg (DE); Douglas, Alan, 81669 München (DE); Vogel, Martin, 69469 Weinheim (DE)
(72) Inventor: Blume, Jürgen, 69231 Rauenberg (DE); Douglas, Alan, 81669 München (DE); Vogel, Martin, 69469 Weinheim (DE)
(74) Representative: Eder, Michael

(57) **Abstract**

The present invention relates to a medical device of an endoscopic type providing a visually observable image of an examination area in an interior of an organism, which is typically used in the medical field for diagnostic purposes. The present invention is intended for the minimally invasive in-situ examination and analysis of organs and tissues preferably in the interior of a living human being or animal.

## Description

### Field of the Invention

The present invention relates to a medical device of an endoscopic type providing a visually observable image of an examination area in an interior of an organism, which is typically used in the medical field for diagnostic purposes. The present invention is intended for the minimally invasive in-situ examination and analysis of organs and tissues preferably in the interior of a living human being or animal.

### Background of the Invention

During the last decades, such endoscopic devices have gained increasing importance in medical diagnosis of diseases, in particular of injuries, degenerative and tumor diseases. In particular in tumor diagnosis e.g. of the colon colonoscopic examination by endoscopes has become the standard diagnostic method.

A typical prior art endoscope comprises at least:
- a rigid or flexible tube, which is inserted into the interior of the organism and advanced to the organs and/or specific tissues to be examined (examination area);
- a light delivery system to illuminate the examination area, i.e. the organ, tissue or object under inspection. The light source is normally outside the body and the light is typically directed to the examination area via an optical fiber system extending through the rigid or flexible tube;
- an optical and/or optoelectronic system (imaging unit) which gathers and transmits the endoscopic image of the interior of the organism to the viewer from the endoscope, typically a medical professional who uses this endoscopic image for his/her diagnosis;
- an additional channel (working channel) to allow for example the entry of surgical or other medical instruments or manipulators for example for taking tissue samples which can be examined and analyzed histologically or histochemically in a laboratory after the endoscopic intervention.

Endoscopes can generally be divided into two parts, one part - subsequently called "nosepiece", comprising a substantial part of the rigid or flexible tube - which is introduced into a natural or artificial cavity or orifices of the organism, whereas the remainder of the device - subsequently called "eyepiece" - stays outside of the organism and is typically fixed e.g. to a laboratory rack or other type of support. Usually, amongst others, light sources, optical components for the spectral manipulation or analysis of light, devices for processing the electronically converted image and the sensor signals and other components - which cannot reside in the nosepiece - are accommodated in the eyepiece. Nosepiece and eyepiece are in constant physical connection with each other and typically electrical and/or optical signals are exchanged between both parts.

The diameter of the nosepiece is typically small (usually in the range of a few millimeters) and outer shape and dimension of the nosepiece is designed to fit into said cavities or orifices in order to prevent or minimize lesions. The image acquisition at the tip of the nosepiece and the image transmission to the eyepiece can occur optically (by means of an imaging optics projecting the image of the area under examination (examination area) onto the aperture of an optical fiber bundle wherein each fiber of the optical fiber bundle gathers and transmits one pixel of this image to the eyepiece, or by means of a lens system distributed over the length of the nosepiece typically producing a plurality of virtual intermediate images thus bridging the distance from the tip of the nosepiece to the eyepiece) or otherwise opto-electronically (by means of a sensor array, e.g. a CCD sensor array, being located in the tip region of the nosepiece and transmitting the image data electrically to the eyepiece). In most modern endoscopes the optical image is converted to an electronic signal (either in the tip region of the nosepiece or in the eyepiece) which is subsequently electronically processed and displayed by a monitor.

In case of the afore-mentioned colonoscopy, the nosepiece of the endoscope is inserted into the colon thereby providing an image of the intestinal mucosa while the nosepiece is further advanced through or retracted from the colon. The medical professional who is trained to colonoscopic methods, is able to localize and identify suspicious spots in the intestinal mucosa which may indicate a degeneration, neoplasm or cancerous alteration of tissue. To further consolidate this visual diagnosis the medical professional will take tissue samples of the suspicious tissue spots, which are excised by surgical instruments and dispatched via the working channel of the endoscope. After the endoscopic intervention the tissue samples are examined histologically and/or histochemically in a specialized histological laboratory, which may take several days or weeks until the diagnosis can be confirmed or rebutted. In case of a positive histological or histochemical result a second surgical or endoscopic intervention will be necessary.

This time of waiting for the histological or histochemical results and the final diagnosis and the thereby imposed uncertainty about the findings and the potential necessity of a second surgical or endoscopic intervention are very stressful for the patient. In some cases, the loss of time for confirming the diagnosis required by the examination in the histological or histochemical laboratory may be harmful if the tumor is fast-growing and further proliferates and spreads. As an additional aspect, the classical endoscopic examination techniques require of the medical professional a long training and practice in this field which implies that the reliability and accuracy of the diagnoses largely depends on the experience of the medical professional. Thus, a medical professional who is not experienced in this field may produce a significant percentage of wrong-positive and wrong-negative diagnoses.

### Description of the Invention

It is therefore an object of the present invention to provide an endoscopic device and a corresponding process which allows a quick and reliable in-situ diagnosis of tissue alterations as well as of diseases and conditions of organs and/or tissues under examination.

For the endoscopic device, this object is achieved by an endoscopic device comprising
- an imaging unit for the generation of a visually observable image of an examination area in an interior of an organism, and
- a bioanalytical unit for the qualitative and/or quantitative analysis of at least one physiological or pathological parameter in a definable area of interest within the examination area, preferably by means of an in-situ sensor and/or by means of spectroscopy,
- wherein the area of interest for the qualitative and/or quantitative analysis of the at least one physiological or pathological parameter can be defined under visual control with the aid of the generated visually observable image.

Concerning the endoscopic process, the object is solved by an endoscopic process comprising the steps of:
- generating a visually observable image of an examination area in an interior of an organism by an imaging unit,
- defining an area of interest within the examination area under visual control with the aid of the generated visually observable image, and
- analyzing at least one physiological or pathological parameter in said definable area of interest by a bioanalytical unit, preferably by means of at least one in-situ sensor and/or by means of spectroscopy.

It is an advantage of the inventive endoscopic device and the inventive endoscopic process that the reliability of diagnoses can be increased independent of the experience of the medical professional carrying out the examination. Furthermore, the repertoire of diagnostic methods is increased and additional diagnostic facilities and options are made accessible. As a further advantage, loss of time by waiting for the histological and/or histochemical laboratory results can be eliminated, which means earlier and more successful treatment of the disease and less stress for the patients. Additionally, a further surgical intervention, another anesthesia and another surgical traumatization of the patient may be avoided. Also the problem of common prior art endoscopy - that the potentially pathological spots identified during the first endoscopic examination and confirmed by the laboratory result have to be re-localized during a second surgical intervention - may be avoided as the endoscopic device and method of this invention offers the possibility of treating pathological alterations immediately after their identification and diagnostic evaluation in a single diagnostic and therapeutic appointment. As a consequence, by the inventive endoscopic device and process costs for the medical treatment can be saved and quality of the medical treatment can be improved.

In a preferred embodiment of the endoscopic device or process, the presence, absence, concentration, and/or modification of at least one biomarker and/or the concentration ratio of at least two biomarkers may be detectable by the bioanalytical unit.
For example it may be possible to detect and measure the concentration of biomarkers being endogenously produced in the body by one or more sensors or transducers or by spectroscopic methods wherein this information revealed by the biomarker is an indicator for a physiological or pathological condition.

An example for such biomarkers may be sTNFR (soluble tumor necrosis factor receptors) that are emitted by the tumor cells to inactivate TNF molecules that originate from the immune system. The presence of sTNFR is therefore a strong hint for cancer, and its concentration is much higher in close proximity to a tumor.

It is further possible to correlate for example the detected concentration of one biomarker to one or more other parameters, or for example to the concentration of a second biomarker, for example by subtracting, dividing or other mathematical calculations of the measured parameters. By this, for example, a baseline or a ratio could be formed, artifacts or natural variabilities of anatomical and/or physiological nature can be eliminated and/or the diagnostic reliability can be increased.

Two (or more) measurements of biomarkers can be carried out either with a one-fiber bioanalytical unit or with a multi-fiber bioanalytical unit, or, due to the modular design of the device, with two (or more) independent bioanalytical units (that might share some instrumentation in the eyepiece).

Thus, by providing a bioanalytical unit which can detect the presence, absence, concentration, and/or modification of at least one biomarker and/or the concentration ratio of at least two biomarkers it is possible to directly analyze endogenous molecules and to use this information for making reliable diagnoses.

In a preferred embodiment of the endoscopic device or process the bioanalytical unit may comprise at least one in-situ chemical or biochemical or immunological sensor, preferably biosensor, or sensor array. This could, for example, be individual sensors or one-dimensional or two-dimensional arrays or configurations of such sensors which may detect and convert a chemical or biochemical or immunological parameter at the tip of the nosepiece into preferably an electrical or optical signal which may be analyzed in the eyepiece. Preferable types of sensors may be electrochemical sensors, piezoelectric sensors, or semiconductor sensors, electrooptical sensors or surface plasmon resonance (SPR) sensors. By such embodiments, the measured parameter can be analyzed quantitatively in a selective spot at the tip of the nosepiece with an increased accuracy.

As an example, it is possible to prepare a glass surface (e.g. at the tip of one or more of the fibers of the bioanalytical unit) with a grid of antibody molecules that specifically bind to certain cancer cells. If cancer cells bind to the prepared surface, this can be revealed by different kinds of spectroscopic methods, e.g. by fluorescence spectroscopy. In this case, the fluorescence signal arises directly from the fiber tip and fluorescence light will couple back into the fiber with a good efficiency and reach the detector unit.

In a preferred embodiment of the endoscopic device or process the bioanalytical unit may comprise an in-situ SPR (surface plasmon resonance) sensor or sensor array. By providing a bioanalytical unit working with the SPR method, it is possible to detect or determine a variety of physiological or pathological parameters for example via appropriate biomarkers e.g. by means of highly selective antibodies with a very high selectivity and reliability.

Such SPR sensor may comprise a glass or transparent resin surface (again either at the tip or in a circumferential area of the fiber, or at a flat beveled part of the fiber side) which surface is coated with a thin (nanometer range) layer of a metal or metal oxide (typically gold ,other noble metals or conducting metal oxides) and a grid of antibody molecules wherein the antibody molecules are designed to bind to cells, cell fragments or molecules of which the concentration is to be measured. Upon binding, the refractive index of the antibody layer may change. As is known in the art about the SPR method, light that is totally reflected at the back side of the metal or metal oxide layer, will also couple to its front side by generation of surface plasmons (evanescent wave coupling) which leads to a partial frustration of the total reflection that can be measured and converted to a measurement value of e.g. cell or molecule concentrations.

In a preferred embodiment of the endoscopic device or process the bioanalytical unit may comprise a spectroscopic analysis device, preferably a Raman spectroscopic analysis device or a fluorescence spectroscopic device. By providing a bioanalytical unit with a spectroscopic analysis device it is possible on the one hand to selectively measure one spot - directly at the end of the nosepiece or distant from the end of the nosepiece in the sample - with an increased sensitivity and accuracy. On the other hand the bioanalytical unit may be designed to capture an image (still image, intervallic image sequence or movie) of a larger area providing for example a two- or three-dimensionally spatially dissolved image of the anatomic structures by spectroscopic imaging methods which allows an overview about the area of interest for assessing the dimension and the borders for example of a pathologic alteration. Such suitable spectroscopic methods may comprise in particular fluorescence spectroscopy (single photon or multi photon), absorption spectroscopy, e.g. infrared spectroscopy, UV/VIS (ultraviolet/visible light) spectroscopy, Raman spectroscopy or SPR spectroscopy.

For example fluorescence spectroscopy can be used with (either intrinsic or extrinsic) dyes where for example heights of characteristic peaks in the emission spectra, shifts in the emission spectra (in particular in the peak region) or ratios of spectral intensities at characteristic wavelengths can be measured and analyzed allowing the calculation of concentrations of said dyes. Some dyes can furthermore be used as molecular probes which monitor the presence, absence, concentration, and/or modification of an effector (for example pH, oxygen concentration, inorganic molecules, organic molecules or biomolecules) which specifically binds to said dye. Other dyes may be used to selectively stain specific tissue structures. Furthermore, differences in autofluorescence spectra may be used to characterize tissue type. It is also possible to use Raman spectroscopy to detect extrinsic markers that are manufactured to bind to cancer cells. Those markers could contain a metal like gold, or heavy water (hydrogen replaced by deuterium), which generates Raman spectra with large peaks that differ significantly from that of the surrounding tissue. E.g., heavy water enriched biomolecules show a distinct peak in the Raman spectrum at about 2100 cm⁻¹ (carbon-deuterium stretch vibration) which does not exist in "normal" biological matter (which show a large peak at about 2850 cm⁻¹ for the carbon-hydrogen stretch vibration).

It is also conceivable to provide two or more bioanalytical units working on the basis of the same or different measuring principles in the endoscopic device measuring different parameters in order to e.g. eliminate or reduce artifacts or natural variabilities or to allow differential diagnoses with increased diagnostic reliability. The bioanalytical units may either be installed permanently in the nosepiece or be introduced temporarily through the working channel e.g. only when needed. In this context, it may also be conceivable to decide during the endoscopic examination whether and which type(s) of bioanalytical unit(s) shall be applied depending e.g. on the discovered alterations or on medical or diagnostic requirements of the application. After completion of the measurement, the bioanalytical unit may be removed from the working channel in order to clear the working channel for other purposes (e.g. for other analytical instrumentation or surgical endoscopic instruments). It is furthermore possible to use several types of bioanalytical units sequentially, or to regenerate or to replace the sensors of the bioanalytical unit after each application or measurement.

In a preferred embodiment of the endoscopic device an agent, preferably a dissolved, emulsified or suspended biochemical, chemical and/or immunological agent and/or micro- or nano-spheres or micro- or nano-particles comprising a biochemical, chemical and/or immunological agent may be applicable, wherein said agent is analyzable by the bioanalytical unit, preferably by the spectroscopic analysis device.

In a preferred embodiment the endoscopic process may further comprise the steps of
- applying an agent, preferably a dissolved, emulsified or suspended biochemical, chemical and/or immunological agent and/or micro- or nano-spheres or micro- or nano-particles comprising a biochemical, chemical and/or immunological agent, and
- subsequently analyzing said agent by the bioanalytical unit, preferably by the spectroscopic analysis device.

Such agent could be a diagnostic agent (for example extrinsic dyes or markers) enlarging the range of applications and measurement possibilities. By applying such agent, the range of detectable substances, parameters, biomarkers or cell types can be increased and reliability and accuracy of diagnosis can be improved.

For example extrinsic markers may be applied to the area of interest before the area is analyzed spectroscopically. In general, extrinsic markers may be used because (i) they share the position with the target (by detecting the marker, the target can easily be identified), providing the advantage that molecules or cells can be measured which otherwise could only difficultly or imprecisely or not at all be measured; (ii) extrinsic markers are under control of the measurement process whereas intrinsic markers are, by their nature, not (e.g., fluctuations within the sample, changes across a population of patients); (iii) biochemical or immunological binding behavior can be engineered for high specificity (e.g. antigen-antibody link) and (iv) extrinsic markers can be engineered for specific spectroscopic properties, e.g. tuned to show certain peaks in fluorescence or Raman spectrum that can be detected reliably and precisely even in low concentrations and in presence of a multitude of other molecules or cells.

Among the preferred extrinsic markers are: (i) fluorescent dyes linked to biomolecules or antibodies that bind to, e.g., either membrane proteins which are specific to cancer cells, or to soluble tumor markers like sTNFR (soluble tumor necrosis factor receptors) molecules mentioned above, or specifically designed markers for Raman spectroscopy as mentioned above or dye molecules with a specific binding site e.g. for inorganic, organic or biochemical molecules; (ii) spheres with a diameter between the low micrometer and the middle to large nanometer range, made from metal or carbon of which the surfaces may for example be modified ("functionalized") so that biomolecules or antibodies can be attached to them during the manufacturing process, wherein the biomolecules or antibodies can be chosen e.g. to bind, again, specifically to cancer cells or soluble tumor markers; (iii) membrane vesicles or micro or nano particles with or without functionalized membrane surface, containing dyes or markers or other agents in the lumen (in case of membrane vesicles) or at their surface; or (iv) dissolved, emulsified or suspended dyes or markers.

In a preferred embodiment the endoscopic device may further comprise a drug application unit for the controllable release of a defined quantity of a diagnostic and/or therapeutic drug or other agent into a defined diagnostic and/or therapeutic target area.
In a preferred embodiment the endoscopic process may further comprise the step of releasing a defined quantity of a diagnostic and/or therapeutic drug or other agent into a defined diagnostic and/or therapeutic target area by a drug application unit.

By using such drug application device, a defined quantity of a diagnostic and/or therapeutic drug or other agent can be applied specifically to a defined diagnostic and/or therapeutic target area, minimizing the contamination of surrounding tissues and the impact on the whole organism. Thus, a diagnostic agent (e.g. an extrinsic marker or a dye or other substance) as mentioned above, or a therapeutic agent can be dispensed pinpointedly to the defined diagnostic and/or therapeutic target area that is to be diagnosed or treated, thereby potentially achieving a better diagnostic result (e.g. by reducing the background signal) and less exposure of surrounding tissue and the whole organism to the applied agents (for example in case of using toxic dyes or chemotherapeutics). This effect may even be improved when using agents with functionalized surface regions, as explained above, which specifically detect and attach to their targets.

Such drug application unit typically comprises three parts: (i) One or more reservoirs in the eyepiece of the device where the diluted ready-to-use diagnostic or therapeutic drug may be stored under controlled environment conditions (cooling, light, gas atmosphere, movement e.g. for holding colloids in suspense). (ii) One or more pumps or micro-dispensers (and optionally valves) for conveying a defined quantity of the drug wherein the pump(s) are connected to at least one reservoir and (iii) one or more hollow, preferably flexible tubings connected to the pump(s) that span substantially over the whole length of the nosepiece alongside with the imaging fiber bundle from the eyepiece to an outlet of the tubing in the nosepiece tip and through which the drugs can be delivered. Optionally piezo or other types of micro-dispensers instead of or in addition to the pumps may be localized for example in the tip of the nosepiece. It is also possible to provide the flexible tubing with a remotely controlled movable or extendable outlet nozzle with or without a valve that can be moved to the desired point of application for the drug. In some application cases, it can be favorable not to install the tubing permanently inside the nosepiece, but to introduce and advance the tubing in the working channel of the nosepiece only temporarily for the purpose of drug application. By this the tubing can be moved into and out of the nosepiece tip, e.g., by simply pushing and pulling its eyepiece end. This flexibility has the advantage that the tip of the tubing can be adjusted and monitored by the imaging unit before the drug is applied.

In a preferred embodiment of the endoscopic device or process, the therapeutic agent may comprise a drug being bound to or contained in a drug carrier or an activatable inactive drug. By such embodiment it is possible to bring the inactive diagnostic or therapeutic drug to the intended diagnostic or therapeutic target area prior to releasing or activating the drug in the target area for diagnosis or treatment which may result in a better diagnostic result (e.g. by reducing the background signal) and less exposure of surrounding tissue and the whole organism to the applied agents (for example toxic dyes, chemotherapeutics).

For example it is possible to bind biochemical molecules or particles to one or more drug molecules whereby the drug molecules are rendered inactive. Another binding site of the biochemical molecule or particle may be "functionalized" so that it may bind to specific cells (e.g. cancer cells) or to another biomolecule or antibody that again either binds specifically to the tissue to be treated (e.g. cancer cells), or binds to an active transport system in cell membranes. As another preferable possibility the drug molecules may be confined (either morphologically, or just chemically inactivated) in the interior of a "box" or "cage" like particle or in a membrane vesicle. Depending on the particle design and manufacturing process, the outside can again be functionalized with other biomolecules or antibodies that help to bring the drug molecules to the specific target of application. In both cases, the inactive drug may be linked or fixed almost completely for example to the target cells prior to being activated. This activation may be induced by irradiation with light or other electromagnetic radiation, by excitation of mechanical vibrations of the box or cage molecule (e.g. surface plasmons), or by chemical reactions.

A potentially huge advantage of such a local drug application is that the total dose of the drug application, related to the whole organism (e.g. the human body), is relatively small and specifically localized to the pathologic tissue whereas healthy tissue is spared out. That fact could allow the application of some highly effective drugs for local application that have been ruled out for "global" chemotherapy due to their side effects when systemically applied in therapeutically effective doses.

In a preferred embodiment of the endoscopic device or process the therapeutic drug may be releasable or activatable by irradiation with laser light.
This represents a very effective method for specifically releasing or activating the inactivated drug in the target area.

The "box" or "cage" like particle or the membrane vesicle can be designed so that they break apart upon irradiation with light of a certain wavelength that is tuned to an absorption peak of the box or cage or vesicle. Preferably the "box" or "cage" like particles or the membrane vesicles exhibit a high peak in their optical absorption spectrum in a spectral range where cells and intrinsic biomolecules have low absorption.

It is feasible to use either the laser spot of the bioanalytical unit but typically with higher intensity, or a specific second laser unit which is not used for bioanalytical detection, wherein the laser spot for the drug activation or drug release to the therapeutic target area may be adjusted under visual control of the imaging unit.

In a preferred embodiment the endoscopic device may further comprise a therapeutic unit for treating and/or destroying and/or removing tissue from the therapeutic target area, preferably by releasing or activating a therapeutic drug in a therapeutic target area through irradiation with laser light, and/or by means of a surgical laser.

In a preferred embodiment the endoscopic process may further comprise the steps of
- defining a therapeutic target area and
- treating and/or destroying and/or removing tissue from the therapeutic target area, preferably by means of releasing or activating a therapeutic drug in a therapeutic target area through irradiation with laser light, and/or by means of a surgical laser.

By such embodiment, for example, a pathological or cancerogenous tissue can be treated immediately in connection with the first diagnostic intervention directly through the endoscopic device, so that in many cases another surgical invasion and a loss of time can be avoided.

This treating and/or destroying and/or removing tissue may be achieved chemically by drug release to the therapeutic area as described above. Furthermore classical surgical methods (e.g. using endoscopic surgical instruments or thermocauter) may be applied. As a further possibility a "surgical" laser capable of cutting biological tissue may be provided in the endoscopic device. Unlike all other laser sources mentioned above that are usually low to medium average power cw (continuous wave) lasers, pulsed laser sources with high average power and medium to low repetition rates are preferably used for this purpose.

Technically, it may be feasible, however, to use a common laser source for surgery and spectroscopic measurements and/or release of drugs, wherein the output power is attenuated when the laser is used for spectroscopic measurements or release of drugs.

It may be furthermore advantageous to apply nanoparticles by the drug application device to the area to be treated prior to laser surgery. These nanoparticles may exhibit an absorption spectrum with a high absorption peak in a spectral range where cells and natural biomolecules have low absorption. Furthermore, the surface of the particles may be functionalized to attach to cancer cells as described above. Upon laser irradiation with the wavelength of an absorption peak of the nanoparticles the energy is absorbed by the particles which heat up and destroy the tissue around them whereas the tissue without particles remains without harm (the particles effectively act as laser cutting agent). In cases of certain tumors, it may be favorable to combine local drug application and laser treatment in order to increase the success rate and to minimize side effects for the patient. As a further aspect, it may be possible to control the therapeutic result by the bioanalytical unit immediately after the treatment.

In the following the invention is further explained by means of exemplary embodiments in conjunction with the accompanying drawing, in which

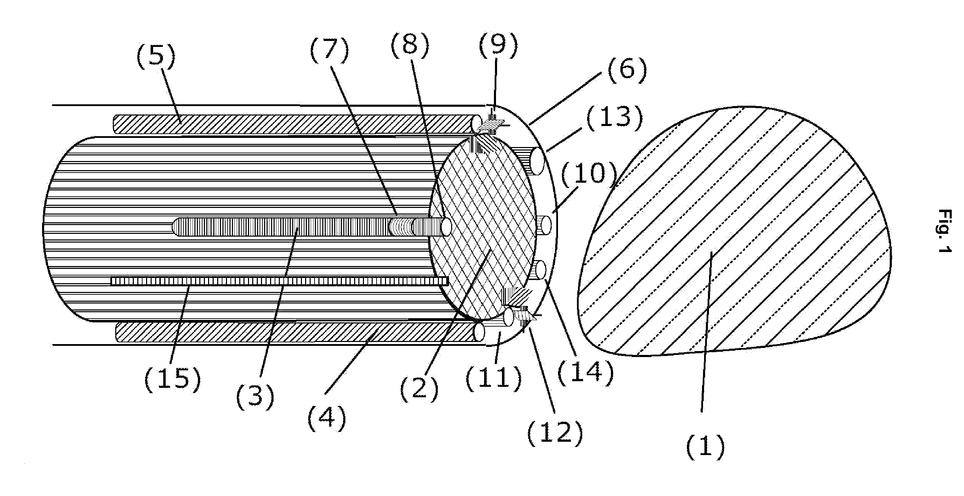

Fig. 1 shows a schematic diagram of the tip of the nosepiece according to an embodiment of the present invention.

As is typical for endoscopes, the endoscopic device of this embodiment is divided into two parts or portions: the flexible nosepiece which is introduced into a natural or artificial cavity or orifices of a human body, and the eyepiece.

As can be seen in Fig. 1, the nosepiece comprises an optical fiber bundle 2 for transmitting the endoscopic image of the examination area 1 in the interior of the human body which image is projected by a collimating lens to the aperture of the optical fiber bundle 2. Each fiber of the optical fiber bundle 2 gathers and transmits one pixel of this image from the tip of the nosepiece to the eyepiece (not shown) where the image is converted to electrical signals by a light sensitive area sensor, like, e.g., a CCD or CMOS sensor. This optical fiber bundle 2 is coaxially surrounded by an outer protection shell 6 which is radially spaced apart from the optical fiber bundle 2. In the space between the optical fiber bundle 2 and the outer protection shell 6 several thin tubular structures are arranged which also substantially span from the tip of the nosepiece to the eyepiece:
(i) Reference sign 3 denotes the optical fiber 3 of the SPR sensor which is part of the bioanalytical unit and analyzes at least one physiological or pathological parameter (e.g. a tumor marker). The cladding of the optical fiber 3 is removed in an annular region 7 near the end of the optical fiber 3 and replaced by a gold coating decorated with linker molecules (e.g. antibodies for the tumor marker). Light that is coupled into the optical fiber at the eyepiece will be transmitted to this coated region where it is totally reflected by the inner side of the gold coating, giving rise to an SPR signal generated upon binding of respective molecules to the linker molecules at the outer surface. The end face 8 of the fiber is reflective so that the light is returned to the eyepiece where the SPR signal can be analyzed. Typically such SPR sensor must be replaced after each measurement. To achieve this, the whole optical fiber may be removed and a new fiber with a readily prepared SPR sensor surface may be inserted.
(ii) Reference sign 4 marks a flexible tubing of the drug application unit by which e.g. dyes, marker solutions or therapeutic drugs can be applied selectively to a desired target region as described above. This flexible tubing of the drug application unit is advanceable and retractable in a separate channel formed in the nosepiece.
(iii) Reference sign 5 denotes an optical fiber for optical scanning spectroscopy which can be used for example for conventional reflection spectroscopy, for fluorescence spectroscopy and for Raman spectroscopy: The laser light of a spectrally tunable continuous wave or pulsed laser transmitted by the optical fiber 5 is focused by a lens and deflected e.g. by a small micro-optical electromechanical system (MOEMS) device comprising a prism and two adjustable micro-mirrors 9 so that the focused laser beam can raster scan the whole area of interest. The spectroscopic signals are collected and returned for measurement either via the same optical path as the excitation light, or via the optical fiber bundle 2.
(iv) Reference sign 10 identifies a small optical fiber bundle 10 of an in-situ sensor (chemo-optical sensor) with several optical fibers of which the end faces are coated with fluorescent molecular probes monitoring the presence, absence, concentration, and/or modification of an effector (for example pH, oxygen concentration, inorganic molecules, organic molecules or biomolecules) which specifically interacts with said fluorescent molecular probe, or with fluorescent antibodies e.g. for biomarkers, wherein each of the fibers is coated with a different type of molecular probe or antibody. Excitation light of a laser source, or a laser diode or a LED (light emitting diode) is guided from the eyepiece through each optical fiber and stimulates fluorescence of the molecular probes or antibodies depending on the presence, absence, concentration, and/or modification of the monitored molecule. The fluorescence signal returns to the eyepiece via the same optical path as the excitation light where this signal is analyzed.
(v) Reference sign 11 indicates an optical fiber 11 for the surgical laser with focusing lens and a device 12 for directing and adjusting the focused laser spot to the tissue to be destroyed.
(vi) A working channel 13 admitting the temporary introduction e.g. of different surgical instruments or analytical equipment or probes of the bioanalytical unit,
(vii) a gas insufflator tube 14 for inflating hollow organs and cavities in the body, an irrigator tube for rinsing the examination area with physiological saline, a suction pump for removing mucus, diagnostic or therapeutic drugs, or blood or tissue debris and a coagulator for hemostasis (all not shown) may optionally be present in the nosepiece.
(viii) Optionally chemo-electrical sensors or transducers 15, such as electrochemical sensors, piezoelectric sensors or semiconductor sensors, measuring further chemical or physical parameters may be accommodated in the tip of the nosepiece and electrically connected to evaluation electronics in the eyepiece.

The structures mentioned above, in particular sensory equipment or drug application tubes may be present once, severalfold or absent in the endoscopic device. Preferably the endoscopic device comprises one or more bioanalytical units which offer a plurality of complementary analytical methods which may help to reduce or eliminate artifacts and wrong-positive and/or wrong-negative diagnoses.
Furthermore, by this approach reliable differential diagnoses may be rendered possible. The skilled person will recognize immediately that the structures or modules described above (items (i) to (viii)) are optional (meaning that they may be present once, severalfold (e.g. different types of sensors) or absent) and merely represent an exemplary combination that may be adapted e.g. to the medical and diagnostic requirements of the intended application by e.g. selecting an appropriate combination of the required or useful modules.

The eyepiece substantially comprises the large and bulky or heat producing components of the imaging unit and the bioanalytical unit which cannot be accommodated inside the nosepiece. Amongst others, the eyepiece of this embodiment contains e.g. the light sources (a gas discharge lamp or a LED (light emitting diodes) or LED arrays for illuminating the bright field endoscopic image, one or more spectrally tunable continuous wave or pulsed lasers for spectroscopic analysis and for activating or releasing drugs in the diagnostic or therapeutic target area and a pulsed surgical laser source for cutting and ablating pathological tissue). Furthermore, the eyepiece contains the opto-electronic sensor converting the optical endoscopic image transmitted by the optical fiber bundle into an electronic signal which is digitally enhanced and analyzed by an image processing unit prior to being displayed preferably in real time on a monitor and optionally parallelly recorded on a mass storage device such as a hard disk. The eyepiece typically also contains a spectral analyzing device for analyzing the signals of the scanning optical spectroscopy. Additionally the eyepiece accommodates optical sensors and analysis electronics for analyzing the optical signals of the chemo-optical transducers and the SPR sensors. Besides, drug reservoirs and pumps for the drug application device are also located in the eyepiece.

In an alternative embodiment, the nosepiece of the endoscopic device has a smaller diameter and is designed to enter the working channel of a "conventional" medical endoscope. With this approach, two imaging units (one of the conventional endoscope and one of the endoscopic device) can be used in parallel. Such embodiment may even be more advantageous than using a stand-alone endoscopic device, as the medical professional can work with the endoscope he is familiar with (conversant with the endoscope handling) and is supplied with the well-known information, but supplementary with additional, independent information. Moreover the medical professional can, e.g., control the current position of the nosepiece within the field of interest. As a further positive aspect, most doctor's offices and hospitals are already equipped with several, specifically adapted medical endoscopes which are chosen and applied depending on the diagnostic task, organ system and position of the area to be examined and on the body size of the patient (e.g. adults or children). It is readily apparent that this type of endoscopes can be used with a variety of conventional endoscopes by which prime costs for buying several endoscopic devices with extensive bioanalytical facilities can be saved. In a further alternative embodiment the bioanalytical unit is not fixedly integrated into the nosepiece but is advanced only temporarily through the working channel offering the possibility to choose the bioanalytical module depending on the medical task or to add further bioanalytical modules at a later time for completing the diagnostic facilities (depending on the available budget or the technical development of improved analytical modules). As a further option, it is conceivable to transmit the digital image data of the endoscopic image together with the analytic results of the bioanalytical unit to a remote medical competence center (e.g. via a secured internet connection) which may assist a less experienced medical professional or a medical professional who is not trained in this method (e.g. in emergency departments, field hospitals, hospitals in developing countries), in making complicated differential diagnoses or discussing difficult medical decisions with a second expert, for example when deciding for an immediate chemotherapeutic or surgical intervention by the means (e.g. drug application unit, surgical laser) provided by this endoscopic device.

In the following some general considerations are given concerning further preferred embodiments of the endoscopic device according to this invention:

The endoscopic device generally comprises at least one imaging unit and at least one bioanalytical unit. The imaging unit relays an image (still image, intervallic image sequence or movie) of the examination area in the interior of the organism to the eyepiece, where the image is typically converted by a camera (e.g. with CCD or CMOS sensor) and electronically processed and displayed on a monitor for observation by the medical professional. The imaging unit usually comprises a collimating lens at the nosepiece end projecting an image of the examination area to the aperture of a coherent bundle of optical fibers within the nosepiece for transmitting the signal light to the eyepiece. Instead of the optical fiber bundle, the image transmission may as well be realized with a chain of relay optics made from, e.g., GRIN (gradient index) lenses distributed over the length of the nosepiece.

The imaging unit (or some structural elements of it) may serve (or contribute to) four purposes:
(i) First, the imaging unit generates a visually observable image (or a movie) of the examination area within the human body, e.g. under white light illumination, that is used by the medical professional to orientate himself or herself and to allow a medical examination of the area by eye.
(ii) Second, the imaging unit may be provided with facilities for generating a visually observable image of the examination area by means of other contrast methods, such as dark field, polarization, phase contrast, differential interference contrast (DIC), confocal scanning methods, or spectral analysis methods which may allow or increase visibility of certain details in the examination area and/or improve diagnostic assessment. Furthermore, other optical methods like widefield fluorescence, Raman scattering, or nonlinear effects like multi photon fluorescence, fluorescence lifetime measurement, or harmonic signal generation imaging may also be implemented in an imaging mode of operation provided by the imaging unit. In particular in case of Raman spectroscopy, but also in some cases of fluorescence spectroscopy, high excitation light power densities are necessary for sufficient signal generation so that preferably one or more laser spots raster scan the examination area, wherein the final observable image is electronically recomposed from the gathered signal of the individual raster scanned pixels. Such image generation by other contrast methods may help the medical professional to recognize different tissue types or identify pathological alterations of tissue, wherein the medical professional may choose one or combine several image generation methods which fit best for the specific diagnostic requirements.
(iii) Third, the imaging unit may be used to control the area of interest (which may be a fixed or freely definable part of the examination area or may comprise the whole examination area) within the area of examination in which the bioanalytical unit is carrying out a measurement of physiological or pathological parameters. When using spectroscopical methods for the measurement of physiological or pathological parameters it may simply be possible to visually observe the spot of focused light of the excitation light source in the examination area by the endoscopic image of the imaging unit. When applying sensor probes (e.g. chemo-optical or chemo-electrical sensors or SPR sensors), the imaging unit may display the tip of the sensor probe within the endoscopic image of the imaging unit whereby the medical professional is capable to localize and adjust the measuring spot of this sensor in relation to the anatomical structures of the examination area.
(iv) Finally, some structural components of the imaging unit may be used to transmit excitation light and recollect signal light for the spectroscopic analysis of a physiological or pathological parameter carried out by the bioanalytical unit. This could be realized as described below:

Excitation light source for the spectroscopic analysis (for example a laser source, a laser diode, a light emitting diode (LED), an arc lamp or gas discharge lamp with or without bandpass filter or monochromator), light detector (spectrally integrating detector like, a photomultiplier tube or a photo diode or a spectral analyzing detector, such as a light spectrometer), and conventional or dichroic beam combiners and splitters - all being part of the bioanalytical unit - are accommodated within the eyepiece. The excitation light is coupled by the conventional or dichroic beam combiner into one or a few or all fibers of the optical fiber bundle of the imaging unit and projected to the area of interest. The collected signal light evoked by the excitation light is again transmitted via the optical fiber bundle of the imaging unit and coupled out by the beam splitter and supplied to the light sensor of the bioanalytical unit which resides within the eyepiece. Alternatively the excitation light may be transmitted via a separate optical path, e.g., a single-mode optical fiber preserving the ability to generate a tightly focused laser spot, or a multimode fiber or a liquid light guide, which can be mounted inside the nosepiece in parallel with the optical fiber bundle of the imaging unit.

Amongst others, the bioanalytical unit may operate in two different spectroscopic modes, both essentially relying on the same spectroscopic methods: (i) in a sample mode, a physiological or pathological parameter is analyzed in a two- (or three-)dimensional area or volume of the sample (analyzed molecules are distributed in the sample volume which is captured), and (ii) in a sensor mode, the physiological or pathological parameter is measured directly at an active surface of the sensor e.g. by a chemo-optical or SPR sensor at a selective spot of the sensor mounted at the tip of the nosepiece (analyzed molecules are interacting with the active surface of the sensor).

(i) In the sample mode, the excitation light exits from the fiber, potentially passes some collimation lens and is directed into the sample area (or volume), for example in the center of the field of view of the imaging unit. This can be accomplished for example with mirrors that are fixedly mounted at the nosepiece tip, so that the light spot position remains constant with respect to the nosepiece tip, and the area of interest is adjusted by moving the nosepiece tip. Alternatively, a small micro-optical electromechanical system (MOEMS) device can be used for providing the capability of defining the area of interest independently within the examination area displayed by the imaging unit without moving the nosepiece tip and changing the endoscopic image.

At the sample area (or volume), the excitation light interacts with either intrinsic or extrinsic markers being naturally present in or beforehandly applied to the sample, and which markers are chosen or designed to generate optical signals that depend on the state of a specific physiological or pathological parameter of the sample. One example for such markers may be an extrinsic marker for e.g. a specific biomolecule or immunological target (for example a fluorescent dye linked to an antibody that specifically binds to e.g. tumor cells), wherein upon binding of said extrinsic marker to the appropriate target the fluorescence emission spectrum of the marker is changed. Another example may be an intrinsic marker naturally occurring in the sample which marker may produce Raman shifted signals, being indicative of a change of a certain physiological or pathological condition. A third example may be the spectral reflectivity of the sample that can be used, e.g., for angiogenesis measurement by determining the vascular density in the tissue (being a hint for a tumor), e.g. via analysis of reflection spectra of hemoglobin in blood capillaries.

The optical signals from the sample and/or the intrinsic or extrinsic markers are collected by the optical fiber bundle of the imaging unit, optically analyzed, as mentioned above, and converted into a two- (or three-) dimensional image.

(ii) Spectroscopical methods can also be used in a sensor mode of the bioanalytical unit which measures molecules interacting with the active surface of a sensor and may be carried out by e.g. a chemo-optical or a SPR sensor at a selective measuring spot of the sensor mounted at the tip of the nosepiece. In this case the optical signal generation takes place for example at an active surface of the optical fiber (SPR sensor) or at the fiber tip (e.g. chemo-optical sensor). The signal light is reflected back typically through the same optical fiber as the excitation light and is optically analyzed as a matter of principle in the same way as described for the sample mode.

As is evident to a person skilled in the art , the exemplified embodiments are given for the purpose of illustrating and explaining the invention and shall not be understood in a sense limiting the invention. All specific embodiments described in this application shall be construed as illustrative examples which do not represent the whole scope of the invention, whereas the scope of protection is exclusively defined by the claims. Furthermore, it will be understood by a person skilled in the art that the features of all embodiments disclosed in the dependent claims and in the description may be combined - individually or together with the features of other embodiments - with the endoscopic device or endoscopic process of the invention as far as they are not mutually exclusive for technical reasons.

## Claims

1. Endoscopic device comprising
- an imaging unit for the generation of a visually observable image of an examination area in an interior of an organism, and
- a bioanalytical unit for the qualitative and/or quantitative analysis of at least one physiological or pathological parameter in a definable area of interest within the examination area, preferably by means of an in situ sensor and/or by means of spectroscopy,
- wherein the area of interest for the qualitative and/or quantitative analysis of the at least one physiological or pathological parameter can be defined under visual control with the aid of the generated visually observable image.

2. Endoscopic process comprising the steps of:
- generating a visually observable image of an examination area in an interior of an organism by an imaging unit,
- defining an area of interest within the examination area under visual control with the aid of the generated visually observable image, and
- analyzing at least one physiological or pathological parameter in said definable area of interest by a bioanalytical unit, preferably by means of at least one *in situ* sensor and/or by means of spectroscopy.

3. Endoscopic device or process according to one of claims 1 or 2, wherein by the bioanalytical unit the presence, absence, concentration, and/or modification of at least one biomarker and/or the concentration ratio of at least two biomarkers is detectable.

4. Endoscopic device or process according to one of the preceding claims, wherein the bioanalytical unit comprises at least one in situ chemical or biochemical or immunological sensor, preferably biosensor, or sensor array.

5. Endoscopic device or process according to one of the preceding claims, wherein the bioanalytical unit comprises an in situ SPR (surface plasmon resonance) sensor or sensor array.

6. Endoscopic device or process according to one of the preceding claims, wherein the bioanalytical unit comprises a spectroscopic analysis device, preferably a Raman spectroscopic analysis device or a fluorescence spectroscopic device.

7. Endoscopic device or process according to one of the preceding claims, wherein an agent, preferably a dissolved, emulsified or suspended biochemical, chemical and/or immunological agent and/or micro- or nano-spheres or micro- or nano-particles comprising a biochemical, chemical and/or immunological agent is/are applicable, wherein said agent is/are analyzable by the bioanalytical unit, preferably by the spectroscopic analysis device.

8. Endoscopic device according to one of claims 1 or 3 to 7, further comprising a drug application unit for the controllable release of a defined quantity of a diagnostic and/or therapeutic drug or other agent into a defined diagnostic and/or therapeutic target area.

9. Endoscopic device of claim 7 or 8, wherein the therapeutic drug is releasable or activatable by irradiation with laser light.

10. Endoscopic device according to one of claims 1 or 3 to 9, further comprising a therapeutic unit for treating and/or destroying and/or removing tissue from the therapeutic target area, preferably by releasing or activating a therapeutic drug in a therapeutic target area through irradiation with laser light, and/or by means of a surgical laser.

11. Endoscopic process according to one of claims 2 to 7, further comprising the steps of
- applying an agent, preferably a dissolved, emulsified or suspended biochemical, chemical and/or immunological agent and/or micro- or nano-spheres or micro- or nano-particles comprising a biochemical, chemical and/or immunological agent, and
- subsequently analyzing said agent by the bioanalytical unit, preferably by the spectroscopic analysis device.

12. Endoscopic process according to one of claims 2 to 7 or 11, further comprising the step of releasing a defined quantity of a diagnostic and/or therapeutic drug or other agent into a defined diagnostic and/or therapeutic target area by a drug application unit.

13. Endoscopic device or process of claims 8 or 12, respectively, wherein the therapeutic agent comprises a drug being bound to or contained in a drug carrier or an activatable inactive drug.

14. Endoscopic process of claim 12 or 13, wherein the therapeutic drug is released or activated in a therapeutic target area by irradiation with laser light.

15. Endoscopic process according to one of claims 2 to 7 or 11 to 14, further comprising the steps of
- defining a therapeutic target area and
- treating and/or destroying and/or removing tissue from the therapeutic target area, preferably by means of releasing or activating a therapeutic drug in a therapeutic target area through irradiation with laser light, and/or by means of a surgical laser.
